# EUROPEAN PATENT APPLICATION

(11) **EP 4 074 252 A1**
(43) Date of publication of application: **19.10.2022**
(21) Application number: 20898175.3
(22) Date of filing: 21.01.2020
(51) Int. Cl.: A61B 5/053

(54) **ELECTRODE BAND, ELECTRODE STRUCTURE, FEED LINE, AND ELECTRICAL IMPEDANCE IMAGING DEVICE**

(30) Priority: 10.12.2019 CN 201911258539
(71) Applicant: Beijing Huarui Boshi Medical Imaging Technology Co., Ltd., Beijing 102609 (CN)
(72) Inventor: LIN, Zhichao, Beijing 102609 (CN); ZHANG, Xin, Beijing 102609 (CN); YU, Yang, Beijing 102609 (CN)
(74) Representative: Dr. Weitzel & Partner
(86) International application number: PCT/CN2020/073352
(87) International publication number: WO 2021/114463

(57) **Abstract**

The present invention provides an electrode band, an electrode structure, a feed line, and an electrical impedance imaging device. The electrode band comprises: a band (1) which is elastic and is provided with a first surface (11) and a second surface (12) which are oppositely disposed; and a plurality of electrodes (4) fixed on the first surface (11) and provided with male buckles (43) which penetrate through the band (1) and are exposed on the second surface (12), the male buckles (43) being electrically connected to the electrodes (4). The electrode band of the present invention is simple in structure and low in cost, and can rapidly and conveniently fix the electrodes to the body of an object to be measured, and the fixing efficiency is high; the electrode band is elastic, and when the electrode band is fixed around the thoracic cavity of the measured object, the elastic force of the band is converted into pressing force on the electrodes, so that good contact between the electrodes and the skin of the thoracic cavity can be ensured; and the electrode band and the feed line are provided in a split mode by means of a male buckle-female buckle structure, so that the design of the electrode band is simplified, the feed line can be repeatedly used, and the replacement cost of the electrode band is reduced.

## Description

The present disclosure claims the priority to Chinese Patent Application CN201911258539.6, titled "ELECTRODE BAND, ELECTRODE STRUCTURE, FEED LINE AND ELECTRICAL IMPEDANCE IMAGING DEVICE", filed on December 10, 2019, which is incorporated herein by reference in its entirety.

### TECHNICAL FIELD

The present disclosure belongs to the technical field of biomedical electrical impedance imaging, and in particular relates to an electrode band, an electrode structure, a feed line, and an electrical impedance imaging device.

### BACKGROUND

Electrical impedance tomography (EIT) is a technique for imaging by utilizing the electrical impedance characteristics of tissues and organs of a measured living body. Since the electrical impedance characteristics of tissues and organs (such as in the thoracic cavity) of the living body are significantly different, the images reconstructed by EIT have high contrast. EIT has broad application prospects due to its advantages such as high resolution, low cost, light-weight equipment, no radiation, and non-invasiveness.

In order to perform biomedical EIT, a certain number (e.g. 16) of electrodes need to be arranged around the body of an object to be measured. Among them, two adjacent electrodes are excited in turn, and the potential difference between the remaining electrodes is measured to acquire data for image reconstruction. For this purpose, the electrodes are preferably expected to be arranged around the body of the object to be measured in a simple manner and in good contact with the body.

Currently, there are two methods for fixing the electrodes around the body of the object to be measured, namely, manual fixing and band-aided fixing. In the first method, the 16 electrodes are manually fixed one by one around the body of the measured object, which involves a cumbersome electrode bonding process, a complex electrode feed line, and cannot ensure uniform distribution of the electrodes. In the second method, the 16 electrodes are fixed onto a band, and the band is then fixed around the body of the measured object. In this fixing method, it is hard to keep the electrodes in good contact with biological tissue all the time due to the poor elasticity of the band. In addition, the electrode feed line is complex and inconvenient for operation, and the length of the band needs to be adjusted according to the size of the measured object, resulting in high cost.

### SUMMARY

Accordingly, the technical problem to be solved by the present disclosure is to overcome the above-mentioned technical defects. To this end, the present disclosure provides an electrode band, which is convenient and simple for fixing electrodes around the body of an object to be measured, and is able to keep the electrodes in good contact with the body of biological tissue all the time.

The present disclosure further provides an electrode structure applicable for the above-mentioned electrode band.

The present disclosure further provides a feed line for use with the above-mentioned electrode band.

The present disclosure further provides an electrical impedance imagingdevice including the above-mentioned electrode band and feed line.

The present disclosure provides an electrode band. The electrode band includes:
a band, which is elastic and is provided with a first surface and a second surface which are oppositely disposed; and
a plurality of electrodes fixed on the first surface, and provided with male buckles which penetrate through the band and are exposed on the second surface, the male buckles being electrically connected to the electrodes.

In a preferred solution, the band may be made of one, or a combination of at least two, of an elastic fabric, an elastic silica gel, and an elastic belt.

In a preferred solution, the electrodes may be arranged uniformly or non-uniformly in at least one row on the first surface along a length direction of the band.

In a preferred solution, the electrode band may further include:
a first connecting structure provided on the first surface; and
a second connecting structure, provided on the second surface, and detachably connected to the first connecting structure.

The present disclosure further provides an electrode structure, which is applicable for the electrode band described in any one of the above implementations. The electrode structure includes:
a flexible printed circuit layer;
at least one copper foil layer, attached to a top surface and/or a bottom surface of the flexible printed circuit layer to form an inner core layer;
a male buckle, which is connected and in contact with an outermost copper foil layer of the inner core layer; and
a conductive silica gel, which covers the inner core layer, and exposes the male buckle.

In a preferred solution, there may be two copper foil layers, which may be attached to the top surface and the bottom surface of the flexible printed circuit layer, respectively.

In a preferred solution, the inner core layer may be provided with multiple through holes; and wherein the conductive silica gel covers the inner core layer by casting after being melted into a liquid.

In a preferred solution, the conductive silica gel may have a portion located outside an outer periphery of the inner core layer; and wherein the electrode (4) is fixed onto the band (1) by utilizing a thread that penetrates the band and the portion of the conductive silica gel,.

The present disclosure further provides a feed line. The feed line includes:
an electrode feed line, having one end electrically connected to an electrical impedance imaging terminal and the other end provided with multiple electrode interfaces, where the electrode interfaces are provided with female buckles suitable for being buckled with the male buckles described in any one of the above implementations to realize electrical connection.

In a preferred solution, the electrode feed line may include:
an Type-Ifeed line, having one end suitable for being connected to the electrical impedance imaging terminal and the other end extending and splitting to form two subinterfaces; and
two II-level electrode feed lines, each having one end connected to the subinterface and the other end extending and splitting into two branches, where each branche is provided with at least one electrode interface, and wherein the electrode interface is provided with the female buckle.

The present disclosure further provides an electrical impedance imaging device, including an electrode band, a feed line, and an electrical impedance imaging terminal, where the electrode band is the electrode band described in any one of the above implementations, and the feed line is the feed line described in any one of the above implementations.

In a preferred solution, the electrodes in the electrode band may adopt the electrode structure described in any one of the above implementations.

The technical solutions provided by the present disclosure have the following advantages:
1. The electrode band of the present invention includes a band and electrodes. The band is elastic, and is provided with the first surface and the second surface which are oppositely disposed. The electrodes are fixed on the first surface, and are provided with male buckles which penetrate from the first surface of the band and are exposed on the second surface. The male buckles are electrically connected to the electrodes. The electrode band of the present invention 1) is simple in structure and low in cost, and can rapidly and conveniently fix the electrodes to the body of an object to be measured, and the fixing efficiency is high;. 2) The band is elastic, and when the electrode band is fixed around the thoracic cavity of the measured object, the elastic force of the elastic band is converted into pressing force on the electrodes, so that good contact between the electrodes and the skin of the thoracic cavity can be ensured. The elastic band can also be well adapted to the breathing movement of the measured object, which is beneficial for long-term monitoring and real-time imaging of the thoracic cavity. In addition, due to the appropriate elasticity of the material of the band, the electrodes are uniformly distributed around the thoracic cavity, and all the electrodes are located on the same tomographic surface, which facilitates the construction of an image reconstruction model. 3) The electrode band and the feed line are provided in a split mode by means of a male buckle-female buckle structureso that placing the feed line in the electrode band is avoided, the design of the electrode band is simplified, the feed line can be repeatedly used, the replacement cost of the electrode band is reduced, and the sanitation condition of different patients using the electrode band is improvided.
2. As to the electrode band of the present invention, the electrodes are uniformly or non-uniformly arranged in at least one row on the first surface of the band to facilitate use in different situations.
3. As to the electrode band of the present invention, the first connecting structure is provided on the first surface, the second connecting structure is provided on the second surface, and the first connecting structure and the second connecting structure are detachably connected. When fixing the electrode band to the thoracic cavity of the object to be measured, the first connecting structure and the second connecting structure are connected in cooperation, so as to quickly fix the electrode band to the body of the object to be measured.
4. The electrode structure of the present invention includes a flexible printed circuit layer, copper foil layer(s), a male buckle, and a conductive silica gel. The flexible printed circuit layer, the copper foil layer and the conductive silica gel are flexible and have strong deformability. When the electrode band is fixed to the body of the measured object, the electrode structure can be flexibly fitted to the contour of the body, with a large contact area and desired contact effect. In addition, the male buckle can easily penetrate through the band so as to be connected with the female buckle of the feed line.
5. As to the electrode structure of the present invention, the inner core layer formed by the flexible printed circuit layer and the copper foil layer is provided with multiple through holes. After the conductive silica gel is melted into a liquid, it is casted to cover the inner core layer, so as to form the electrode structure of the present disclosure. The electrode structure has good conductivity, a large contact area, and is not easy to delaminate.
6. As to the electrode structure of the present invention, the conductive silica gel has a portion located outside an outer periphery of the inner core layer, and the electrode is fixed onto the band by utilizing a thread that penetrates through the band and the portion of the conductive silica gel. The thread fixing method is firm, environmentally friendly, and safe.
7. The feed line of the present invention includes a electrode feed line. The electrode feed line has one end configured to be electrically connected to the electrical impedance imaging terminal and the other end provided with multiple electrode interfaces. The electrode interfaces are provided with the female buckles suitable for being buckled with the male buckles to realize electrical connection. The electrode band and the feed line are provided in a split mode, and can be used for many times. The feed line is reusable after the electrode band is replaced, thereby reducing the cost.
8. As to the feed line of the present invention, the electrode feed line includes the Type-I feed line and Type-II feed lines. The Type-I feed line has one end suitable for being connected to the electrical impedance imaging terminal and the other end extending and splitting to form two subinterfaces. There are two Type-II feed lines, each Type-II feed line has one end connected to the subinterface and the other end extending and splitting into two branches. Each branch is provided with at least one electrode interface, and the electrode interface is provided with the female buckle. The design has the advantages of simple structure, convenient storage, flexible length, and easy connection with the electrode band.
   When the electrode band is fixed to the body of the measured object, the two Type-II feed lines are buckled on the electrode band through the male buckles and the female buckles. The operator can disconnect or reconnect the Type-I feed line and the Type-II feed line at any time, without assembling and disassembling the feed line on the measured object for many times.
9. The present invention further provides an electrical impedance imaging device, including an electrode band, a feed line, and an electrical impedance imaging terminal. The electrode band is the above-mentioned electrode band, and the feed line is the above-mentioned feed line. Since the electrical impedance imaging device adopts the above-mentioned electrode band and feed line, it has all the advantages of the above-mentioned electrode band and feed line.
10. As to the electrical impedance imaging device of the present invention, the electrodes in the electrode band all adopt the above-mentioned electrode structure. Since the above-mentioned electrode structure is adopted, they have all the advantages of the above-mentioned electrode structure.

### BRIEF DESCRIPTION OF THE DRAWINGS

In order to illustrate the technical solutions in the specific embodiments of the present disclosure or in the prior art more clearly, the drawings involved in the description of the specific embodiments or in the prior art will be briefly described below.
FIG. 1 is a front view of an electrode band provided by the present disclosure;
FIG. 2 is a side view of the electrode band provided by the present disclosure;
FIG. 3 is a back view of the electrode band provided by the present disclosure;
FIG. 4 is a schematic view of a Type-I feed line provided by the present disclosure;
FIG. 5 is a schematic view of a Type-II feed line provided by the present disclosure;
FIG. 6 is a schematic view of connection between the Type-II feed line and a back side of the electrode band;
FIG. 7 is a schematic view of preparing an electrode and penetrating the electrode onto a band;
FIG. 8 is a schematic view of operation of an electrical impedance imaging system; and

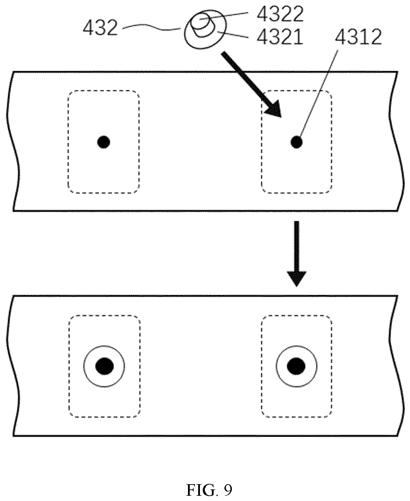

FIG. 9 is a schematic view of assembling an upper cap with a male buckle sewn on the band.

Reference Signs: 1. band; 11. first surface; 12. second surface; 2. first connecting structure; 3. second connecting structure; 4. electrode; 41. flexible printed circuit layer; 42. copper foil layer; 43. male buckle; 431. male buckle base; 4311. sheet-like base layer; 4312. stud body; 432. upper cap; 4321. snap groove; 4322. male buckle stud; 44. conductive silica gel; 45. through hole; 5. electrode feed line; 50. female buckle; 51. Typle-I feed line; 511. subinterface; 52. Type-II feed line; 521. electrode interface; and 6. electrical impedance imaging terminal.

### DETAILED DESCRIPTION OF THE EMBODIMENTS

The technical solutions of the present disclosure will be described in detail below with reference to the drawings.

### Embodiment 1

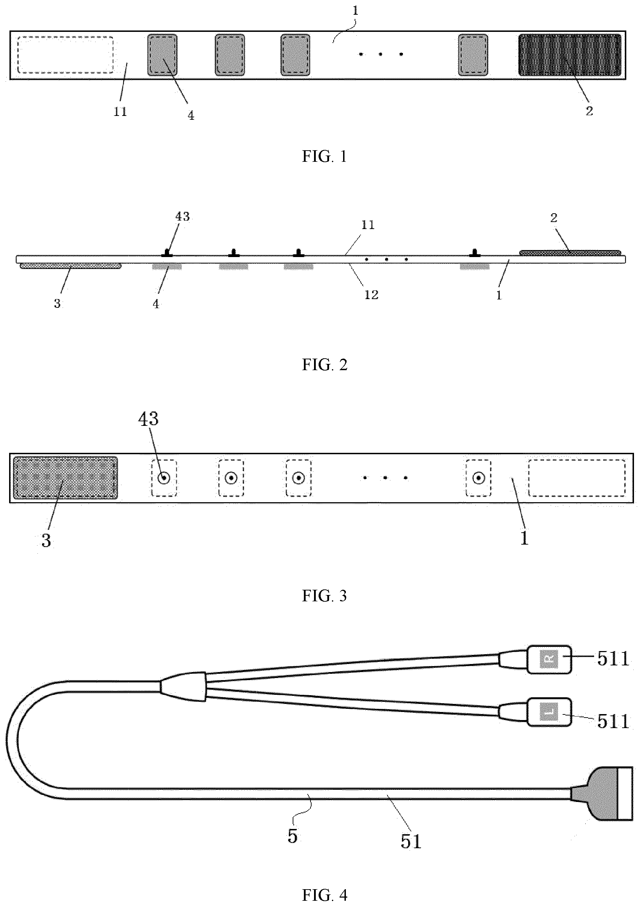

This embodiment provides an electrode band. As shown in FIGS. 1 to 3, the electrode band includes: a band 1, which is elastic and is provided with a first surface 11 and a second surface 12 that areoppositely disposed; and a plurality of electrodes 4 fixed on the first surface 11 and provided with male buckles 43. The male buckles 43 penetrate through the band 1 and are exposed on the second surface 12, and is electrically connected to the electrode 4.

The band 1 is made of an elastic fabric. As a variant design solution, the band 1 may be one or a combination of at least two of an elastic silica gel, an elastic belt, and a rubber belt.

The electrodes 4 are uniformly arranged in a row on the first surface 11 along a length direction of the band 1. According to actual needs, the electrodes 4 may also be non-uniformly arranged in at least one row on the first surface 11.

The electrode band further includes: a first connecting structure 2 provided on the first surface 11; and a second connecting structure 3 provided on the second surface 12 and detachably connected to the first connecting structure 2.

In this embodiment, the first connecting structure 2 is a hook surface of a Velcro, and the second connecting structure 3 is a loop surface of the Velcro, or vice versa. In addition, other detachable connecting structures may also be used.

Specifically, in this embodiment, the electrode band is used for electrical impedance imaging, and is provided with 16 electrodes, which are uniformly distributed on the band 1. An electrode feed line extends at one end to form 16 electrode interfaces for being connected with the 16 electrodes on the electrode band. The electrode interfaces are connected to the electrodes in a snap connection manner (i.e. through the male buckle 43 and the female buckle 50).

In this embodiment, the electrode band has the following advantages. 1) The electrode band is simple in structure and low in cost, and can rapidly and conveniently fix the electrodes to the body of an object to be measured, and the fixing efficiency is high. 2) the band 1 is elastic, and when the electrode band is fixed around the thoracic cavity of the measured object, the elastic force of the band 1 is converted into pressing force on the electrodes 4, so that good contact between the electrodes 4 and the skin of the thoracic cavity can be ensured. The elastic band 1 can also be well adapted to the breathing movement of the measured object, which is beneficial for long-term monitoring and real-time imaging of the thoracic cavity. In addition, due to the appropriate elasticity of the material of the band 1, the electrodes 4 are uniformly distributed around the thoracic cavity, and all the electrodes 4 are located on the same tomographic surface, which facilitates the construction of an image resconstruction model. 3) The electrode band and the feed line are provided in a split mode by means of the structures of the male buckle 43 and the female buckle 50, so that placing the feed line in the electrode band is avoided, the design of the electrode band is simplified, the feed line can be repeatedly used, the replacement cost of the electrode band is reduced, and the sanitation condition of different patients using the electrode band is improvided.

### Embodiment 2

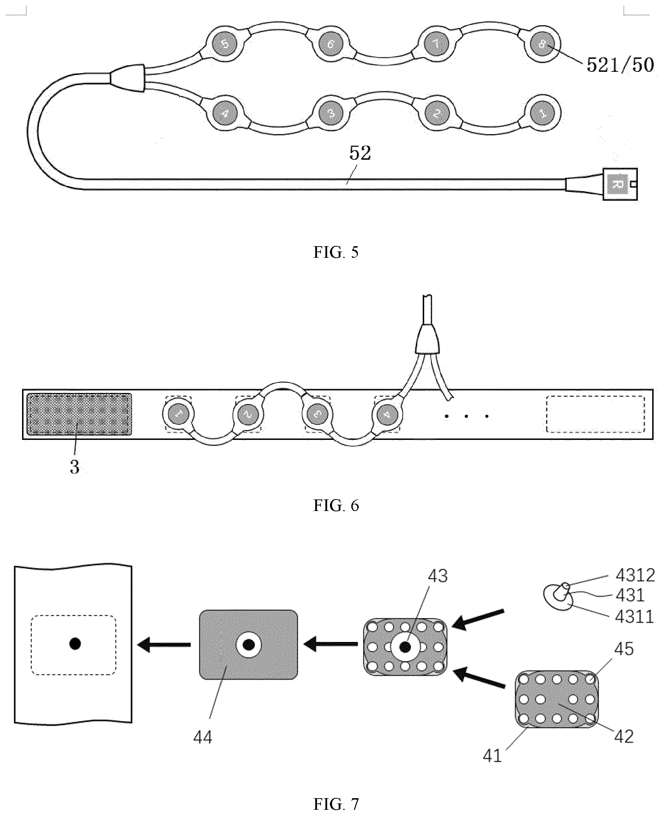

This embodiment provides an electrode structure, which is applicable for the electrode band in Embodiment 1. As shown in FIG. 7, the electrode structure includes: a flexible printed circuit layer 41; two copper foil layers 42, attached to a top surface and a bottom surface of the FPC layer 41 respectively to form an inner core layer; the male buckle 43, which is connected and in contact with an outermost copper foil layer 42 of the inner core layer; and a conductive silica gel 44, which covers the inner core layer and exposes the male buckle 43.

As a variant design solution, there may be one or more than two copper foil layers 42 attached to the top surface and/or the bottom surface of the flexible printed circuit layer 41.

Specifically, the flexible printed circuit layer 41 and the copper foil layer 42 are bonded together by hot-pressing.

In this embodiment, the flexible printed circuit layer 41, the copper foil layer 42 and the conductive silica gel 44 of the electrode structure are flexible and have strong deformability. When the electrode band is fixed to the body of the measured object, the electrode structure can be flexibly fitted to the contour of the body, with a large contact area and desired contact effect. The male buckle 43 can easily penetrate through the band 1 so as to be connected with the female buckle 50 of the feed line.

The inner core layer is provided with multiple through holes 45. After the conductive silica gel 44 is melted into a liquid, it is casted to cover the inner core layer, so as to form the electrode structure of this embodiment. The structure has good conductivity, a large contact area, and is not easy to delaminate.

As shown in FIG. 7, the conductive silica gel 44 has a portion located outside an outer periphery of the inner core layer. A thread penetrates the band 1 and the portion of the conductive silica gel 44, thereby fixing the electrode 4 onto the band 1.

In this embodiment, the conductive silica gel 44 is finally shaped into a rectangle. According to actual needs, those skilled in the art may also shape the conductive silica gel 44 into a circle, a square or other shapes.

The male buckle 43 includes: a male buckle base 431, including a sheet-like base layer 4311 and a stud body 4312 provided on the sheet-like base layer 4311; and an upper cap 432, including a snap groove 4321 and a male buckle stud 4322 opposite to the snap groove 4321. After the stud body 4312 passes through the band 1, the stud body 4312 is detachably snapped into the snap groove 4321, and the male buckle stud 4322 is exposed.

The male buckle 43 may also adopt any other structure available in the prior art.

### Embodiment 3

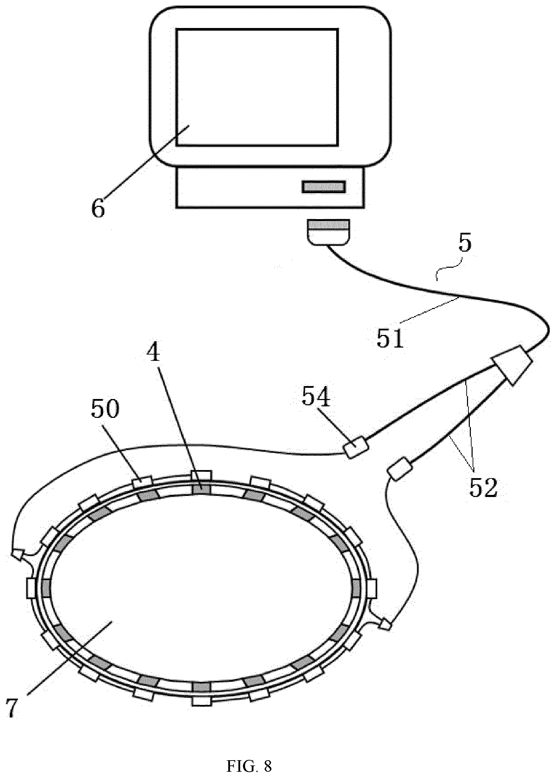

This embodiment provides a feed line. As shown in FIGS. 4, 5, 6, and 8, the feed line includes: an electrode feed line 5, having one end electrically connected to an electrical impedance imaging terminal 6 and the other end provided with multiple electrode interfaces 521. The electrode interfaces 521 each are provided with a female buckle 50, and the female buckle 50 is suitable for being buckled with the male buckle 43 described in Embodiment 1 or 2 to realize electrical connection.

In a preferred solution, the electrode feed line 5 includes: a Type-I feed line 51, having one end suitable for being connected to the electrical impedance imaging terminal 6 and the other end extending and splitting to form two subinterfaces 511; and two Type-II feed lines 52, each having one end connected to the subinterface 511 and the other end extending and splitting into two branches. The two branches each are provided with four electrode interfaces 521, and the electrode interfaces 521 each are provided with the female buckle 50.

As to the feed line in this embodiment, when the electrode band is fixed to the body of the measured object, the two Type-II feed lines 52 are buckled on the electrode band by means of the male buckles 43 and the female buckles 50. An operator can disconnect or reconnect the Type-I feed line 51 and the Type-II feed line 52 at any time, without assembling and disassembling the feed line on the measured object for many times.

### Embodiment 4

This embodiment provides an electrical impedance imaging terminal device, including an electrode band, a feed line and an electrical impedance imaging terminal 6. The electrode band is the electrode band described in Embodiment 1, and the feed line is the feed line described in Embodiment 3.

Since the electrical impedance imaging device adopts the electrode band and the feed line in the above-mentioned embodiments, it has all the advantages of the above-mentioned electrode band and feed line.

Preferably, the electrodes 4 in the electrode band adopt the electrode structure described in Embodiment 2. Since the electrodes adopt the above-mentioned electrode structure, they have all the advantages of the above-mentioned electrode structure.

It is apparent that the above embodiments are merely examples for clear description, and are not intended to limit the implementations. Those of ordinary skill in the art may make modifications or variations in other forms based on the above description. It is unnecessary and impossible to enumerate all the implementations. However, obvious changes or variations made thereto should still fall within the protection scope of the present disclosure.

## Claims

1. An electrode band, comprising:
a band (1), which is elastic and is provided with a first surface (11) and a second surface (12) which are oppositely disposed; and
a plurality of electrodes (4) fixed on the first surface (11) and provided with male buckles (43) which penetrate through the band (1) and are exposed on the second surface (12), the male buckles (43) being electrically connected to the electrodes (4).

2. The electrode band according to claim 1, wherein the band (1) is made of one, or a combination of at least two, of an elastic fabric, an elastic silica gel, and an elastic belt.

3. The electrode band according to claim 1, wherein the electrodes (4) are arranged uniformly or non-uniformly in at least one row on the first surface (11) along a length direction of the band (1).

4. The electrode band according to claim 1, wherein the electrode band further comprises:
a first connecting structure (2) provided on the first surface (11); and
a second connecting structure (3) provided on the second surface (12) and detachably connected to the first connecting structure (2).

5. An electrode structure, applicable for the electrode band according to any one of claims 1 to 4, and comprising:
a flexible printed circuit layer (41);
at least one copper foil layer (42), attached to a top surface and/or a bottom surface of the flexible printed circuit layer (41) to form an inner core layer;
a male buckle (43), which is connected and in contact with an outermost copper foil layer (42) of the inner core layer; and
a conductive silica gel (44), which covers the inner core layer and exposes the male buckle (43).

6. The electrode structure according to claim 5, wherein the copper foil layers (42) have two layers, which are attached to the top surface and the bottom surface of the flexible printed circuit layer (41), respectively.

7. The electrode structure according to claim 5, wherein the inner core layer is provided with multiple through holes (45); and wherein the conductive silica gel (44) covers the inner core layer by casting after being melted into a liquid.

8. The electrode structure according to any one of claims 5 to 7, wherein the conductive silica gel (44) has a portion located outside an outer periphery of the inner core layer; and wherein the electrode (4) is fixed onto the band (1) by utilizing a thread that penetrates the band (1) and said portion of the conductive silica gel (44).

9. A feed line, comprising:
an electrode feed line (5), having one end which is adapted for being electrically connected to an electrical impedance imaging terminal (6) and the other end provided with multiple electrode interfaces (521), wherein the electrode interfaces (521) are provided with female buckles (50) which is adapted for being buckled with the male buckles (43) according to any one of claims 1 to 8 to realize electrical connection.

10. The feed line according to claim 9, wherein the electrode feed line (5) comprises:
a Type-I feed line (51), having one end which is adapted for being connected to the electrical impedance imaging terminal (6) and the other end extending and splitting to form two subinterfaces (511); and
two Type-II feed lines (52), each having one end connected to the subinterface (511) and the other end extending and splitting into two branches, wherein each branch is provided with at least one electrode interface (521), and wherein the electrode interface (521) is provided with the female buckle (50).

11. An electrical impedance imaging device, comprising an electrode band, a feed line, and an electrical impedance imaging terminal (6), wherein the electrode band is the electrode band according to any one of claims 1 to 8, and wherein the feed line is the feed line according to any one of claims 9 to 10.

12. The electrical impedance imaging device according to claim 11, wherein the electrode (4) on the electrode band adopts the electrode structure according to any one of claims 5 to 8.
